# EUROPEAN PATENT SPECIFICATION

(11) **EP 1 384 132 B1**
(45) Date of publication and mention of the grant of the patent: **11.04.2007**
(21) Application number: 03707874.8
(22) Date of filing: 13.02.2003
(51) Int. Cl.: A61B 19/00

(54) **SURGICAL SYSTEM**
CHIRURGISCHES SYSTEM
SYSTEME CHIRURGICAL

(30) Priority: 21.03.2002 US 103382; 16.10.2002 US 272023
(43) Date of publication of application: 28.01.2004
(73) Proprietor: Alcon, Inc., 6331 Hünenberg (CH)
(72) Inventor: BOUKHNY, Mikhail, Laguna Niguel,CA 92677 (US); DACQUAY, Bruno, Irvine, CA 92612 (US); FANNEY, Douglas, M., San Clemente, CA 92673 (US); SOUTHARD, Michael, A., Arlington, TX 76017 (US); THOE, David, Aliso Viejo, CA 92656 (US)
(74) Representative: Hanna, Peter William Derek
(86) International application number: PCT/US2003/004310
(87) International publication number: WO 2003/081379

(56) References cited:
- EP-A- 0 630 820
- JP-A- 9 205 291
- US-A- 4 833 306
- US-A- 4 844 259
- US-A- 5 283 943
- US-A- 5 845 264
- US-A- 5 996 889
- US-A- 6 005 482
- US-A- 6 036 458
- US-A- 6 098 892
- US-B1- 6 341 726

## Description

### Background of the Invention

This invention relates generally to the field of cataract surgery and more particularly to surgical systems used during the phacoemulsification technique of cataract removal.

The human eye in its simplest terms functions to provide vision by transmitting light through a clear outer portion called the cornea, and focusing the image by way of the lens onto the retina. The quality of the focused image depends on many factors including the size and shape of the eye, and the transparency of the cornea and lens.

When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens function by an artificial intraocular lens (IOL).

In the United States, while other lens removal modalities are being introduced, the majority of cataractous lenses are still removed by a surgical technique called ultrasound phacoemulsification During this procedure, a thin phacoemulsification cutting tip is inserted into the diseased lens and vibrated ultrasonically. The vibrating cutting tip liquifies or emulsifies the lens so that the lens may be aspirated out of the eye. The diseased lens, once removed, is replaced by an artificial lens.

A typical ultrasonic surgical device suitable for ophthalmic procedures consists of an ultrasonically driven handpiece, an attached cutting tip, and irrigating sleeve and an electronic control console. The handpiece assembly is attached to the control console by an electric cable and flexible tubings. Through the electric cable, the console varies the power level transmitted by the handpiece to the attached cutting tip and the flexible tubings supply irrigation fluid to and draw aspiration fluid from the eye through the handpiece assembly. The disposable portions of the system, such as the cutting tips, fluid tubings, cassette, drapes and sleeves, are generally sold together as a complete unit in the form of a surgical pak.

In use, the ends of the cutting tip and irrigating sleeve are inserted into a small incision of predetermined width in the cornea, sclera, or other location. The cutting tip is ultrasonically vibrated along its longitudinal axis within the irrigating sleeve by the crystal- driven ultrasonic horn, thereby emulsifying the selected tissue in situ. The hollow bore of the cutting tip communicates with the bore in the horn that in turn communicates with the aspiration line from the handpiece to the console. A reduced pressure or vacuum source in the console draws or aspirates the emulsified tissue from the eye through the open end of the cutting tip, the cutting tip and horn bores and the aspiration line and into a collection device. The aspiration of emulsified tissue is aided by a saline flushing solution or irrigant that is injected into the surgical site through the small annular gap between the inside surface of the irrigating sleeve and the cutting tip.

With the advances that have been made in the last few years in digital circuitry, manufacturers are able to design and built surgical instruments that can automatically change the operating parameters to suit special techniques or situations. Operating parameters such as aspiration fluid flow rate and vacuum, irrigation fluid flow rate and pressure and handpiece power and duty cycle can all be preprogrammed for a specific surgeon or surgical procedure. In addition, the various cutting tips, sleeves, tubings and cassettes can be customized to suit the techniques being used by the surgeon. In order optimize the system, it is important that the operating parameters, tips, sleeves, tubings and cassettes all be designed to work together. With the various disposable products that are available today, it is often difficult for the surgeon to know if the operating parameters of the surgical console have been optimized for the contents of the surgical pak being used.

One prior art device illustrated in U. S. Patent Nos. 5,899, 674 and 6,059, 544 (Jung, et al.) discloses a surgical cassette having an identification system that can be used by the surgical console to identify the type of cassette being used. Another similar device,illustrated in U. S. Patent No. 6,036, 458 (Cole, et al.), discloses a surgical cassette having an identification system that can be used by the surgical console to identify the type of cassette being used as well as how many times the cassette has been used. None of these references discloses a system wherein the surgical console can identify all of items contained in the surgical pak, and automatically adjust the operating parameters of the system for those contents.

EP-A-0,630,820 (C. Stiefenhofer GmbH) describes a system in which sterile surgical packs may be monitored using a barcode read by a barcode reader. Information concerning the sterilisation process is automatically recorded along with batch number which may be used for tracing the provenance and sterile integrity of the contents of the pack, or for inventory management.

Document DE 2 01 00 591 U1 discloses a system comprising the features defined in the preamble of claim 1.

Therefore, a need continues to exist for a system that can identify all of the contents of a surgical pak and automatically adjust the operating parameters of the system for those contents.

### Brief Summary of the Invention

The present invention provides a surgical procedure identification system and software in accordance with claims which follow. The system improves upon the prior art by providing a surgical system console having an electronic identification system, such as a bar code scanner, magnetic reader or other sensing means (e. g., optical, magnetic or other system) system that works in conjunction with a unique identifier on the surgical pak so as to identify the contents of the pak to the surgical console control (e. g., CPU). That information can be used for a variety of further activities, such as inventory management, product usage, traceability, OR support, patient billing, collection of statistical data, automatic setting of the surgical operating parameters by the surgical console, servicing and to print out the contents of the surgical pak on a list or any other recordable media (e. g., DVD, CD-ROM, floppy disk, hard drive, ZIP drive).

Accordingly, one objective of the present invention is to provide a surgical system having a console with an electronic identification system.

Another objective of the present invention is to provide a surgical system having a console capable of identifying the contents of the surgical paks used throughout the procedure.

Another objective of the present invention is to provide a surgical system having a surgical pak having contents that are identifiable to the surgical console.

Still another objective of the present invention is to provide a surgical system wherein information from the contents of the surgical paks is identified to the surgical console, and the console uses this information for setting automatically the operating parameters to be used by the surgical console.

### Brief Description of the Drawings

FIG. 1 is a perspective view of a surgical console that may be used with the present invention, showing the surgical pak in phantom.
FIG. 2 is an exploded perspective view of a surgical pak that may be used with the present invention showing the various contents of the pak.
FIG. 3 is an enlarged perspective view of a surgical pak that may be used with the present invention taken at circle 3 on FIG. 2.
FIG. 4 is a schematic illustration of a surgical system console that may be used with the present invention.
FIG. 5 is an illustration of a printout that may be obtained from the present invention.
FIG. 6 is a schematic illustration of the system of the present invention.

### Detailed Description of the Invention

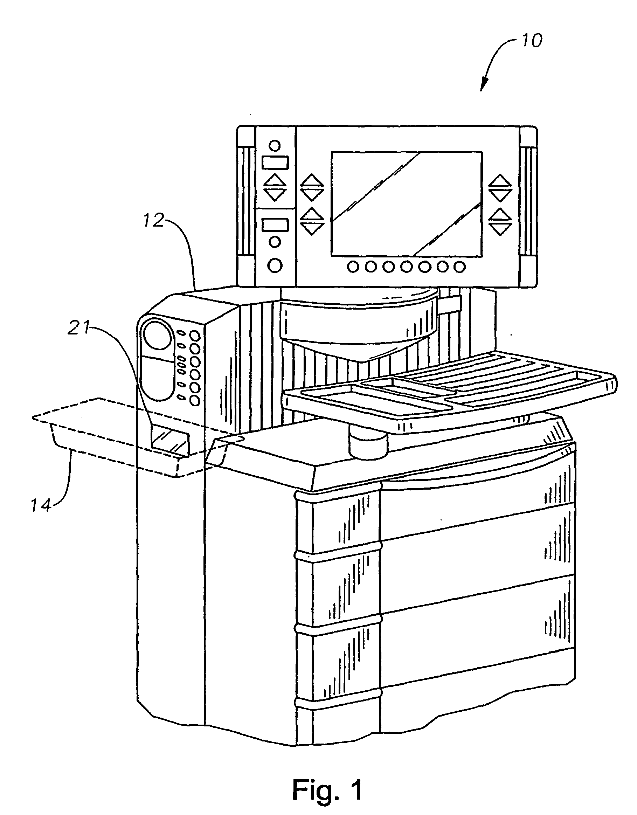

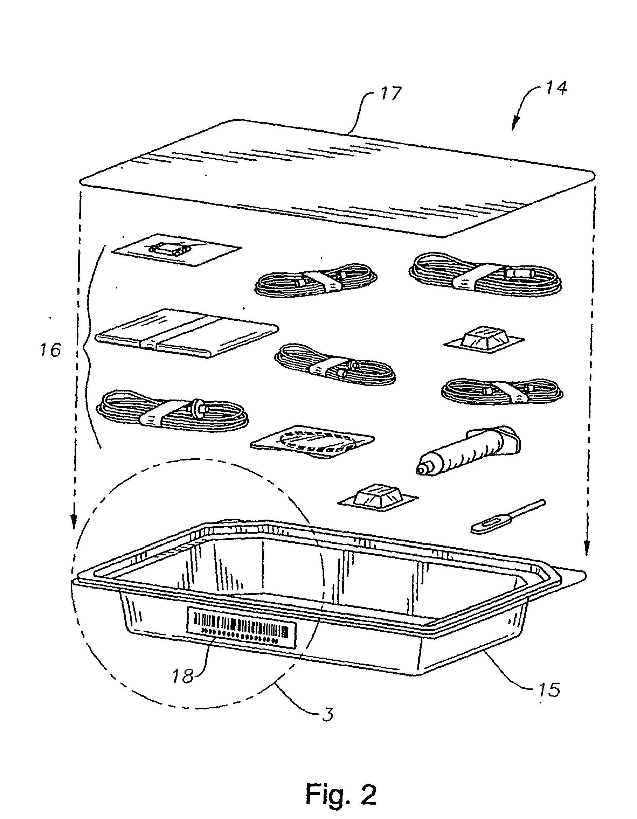

As best seen in FIG. 1 system 10 of the present invention generally includes surgical console 12 and surgical pak 14. Console 12 may be any suitably modified commercially available surgical console, such as the SERIES TWENTY THOUSAND® LEGACY® or ACCURUS® surgical systems available from Alcon Laboratories, Fort Worth, Texas. Pak 14 may be any suitably modified commercially available surgical pak, such as those sold by Alcon Laboratories, Inc., Fort Worth, Texas and, as best seen in FIG. 2, may contain any of a variety of components 16 required to perform a particular surgical procedure, such as cutting tips, sleeves, probes, cassettes, tubing sets, syringes, drapes, etc. Alternative paks 14 may contain pharmaceutical, viscoelastic agents or intraocular lenses. Components 16 are kept sterile in tray 15 by lid 17.

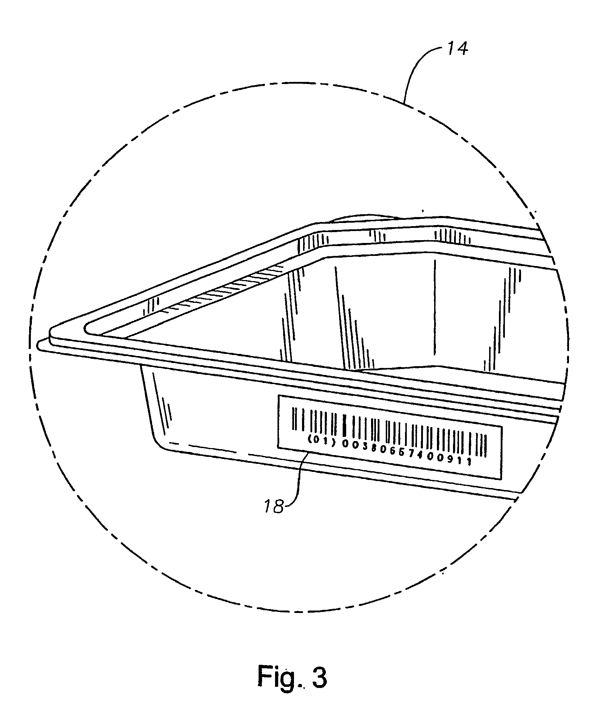

As best seen in FIG. 3, pak 14 contains identification device 18, such as a bar code, that identifies the contents of pak 14. Identification device 18 can either be external, such as with a bar code, or internal, such as with a magnetic device. In use, identification device18 is presented to reader 21 on or connected to console 12. Reader 21 recognizes device 18, identifies pak 14 and components 16 contained in pak 14 and transmits this information to console 12. Console 12 uses this information, under appropriate software control as discussed below, to adjust automatically the operating parameters of console 12 to coincide with components 16 ofpak 14 using factory or user programmable settings.

Device 18 and reader 20 may be any of a variety of suitable electrical, magnetic or optical devices readily commercially available and well-known in the art.

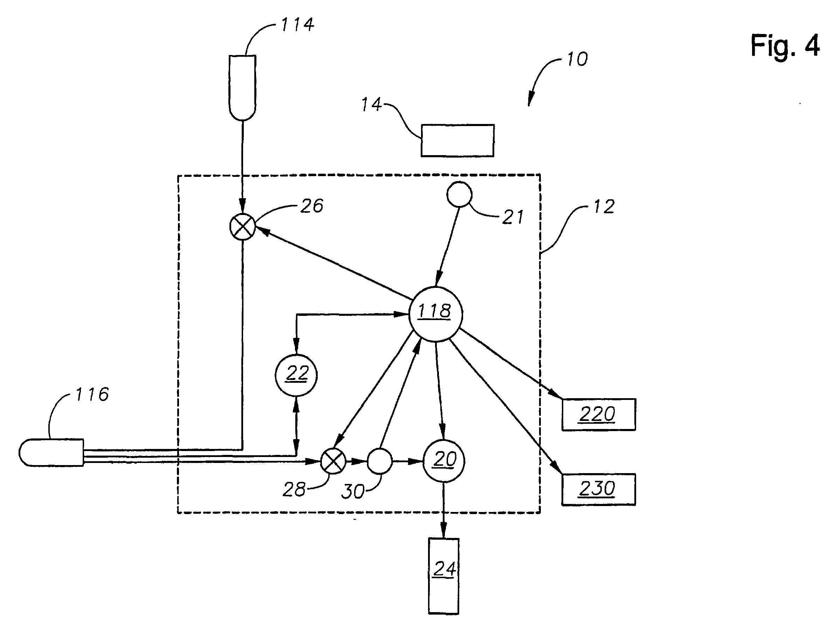

As best seen in FIG. 4, control console 12 generally includes CPU 118, aspiration pump 20, handpiece power supply 22, infusion fluid valve 26, aspiration valve28 and aspiration pressure sensor 30. Information supplied to CPU 118 from reader 21 is used to control aspiration valve 28, pump 20 and infusion fluid valve 26. CPU118 also controls the power supplied to handpiece 116 by power supply 22. Aspirated fluid is directed by pump 20 to collection container 24.

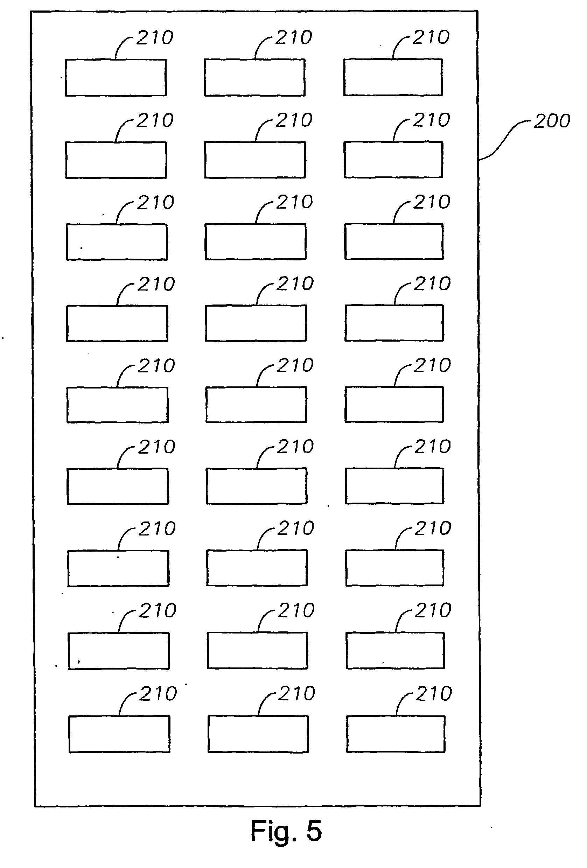

In addition, control console 12 can signal printer 220 to print out a list of the contents of pak or paks 14 used during surgery. As seen in FIG. 5, list 200 may be in the form of a series of self-adhesive labels 210 that can be easily detached from list 200 and placed on the patient's medical chart so as to be able to track the various products, lenses, pharmaceuticals, etc. that were used during the surgical procedure. Printer 220 may communicate with control console 12 either via a hardwire connection, or a wireless connection (e. g. BLUETOOTH®).

System 10 may also be used for inventory control/tracking by providing information regarding paks 14 or components 16 being used by the owners of system 10 to inventory management system or software 230, there being numerous inventory management software programs and systems being commercially available and well-known in the art. Inventory management system 230 can be used to re-order paks 14 and/or components 16 automatically. In addition, information such as the serial numbers, lot numbers or other information regarding paks 14 or components 16 can be tracked with system 10 and/or sent directing to the manufacturer(s) of paks 14 and/or components 16 through inventory management system 230. Inventory management system 230 may communicate with control console 12 either via a hardwire connection, or a wireless connection (e. g. BLUETOOTH®).

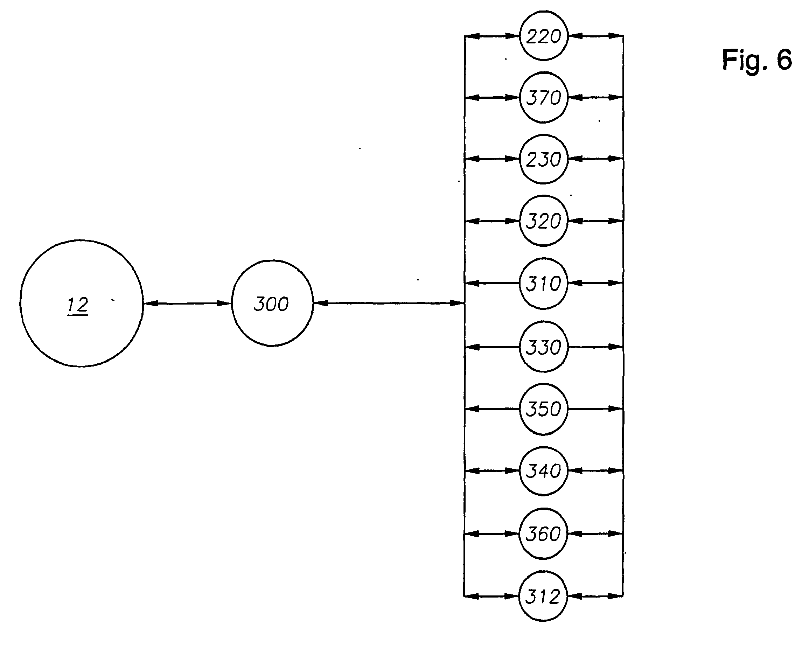

As seen in FIG. 6, system 10 may contain appropriate hardware 300 (e. g. , modem, ethernet card) and software to as to be electronically connected to communications devices external to console 12 within or outside of the surgical center, such as intranet or local area network (LAN) server 310, personal digital assistant (PDA) 320 or internet server 330, so as to provide a wide variety of information about the operation of console 12 and/or the surgical procedure (s) performed by console 12. Statistical data (e. g., patient data, performance parameters, surgical operating parameters (e. g., vacuum levels, ultrasound power, irrigation pressure), a video record of a surgical operation, or surgical operating times) may be transferred between remotely located console 12 and console 312, console 12 and surgical center database 340, and/or console 12 and manufacturer 350 of console 12 or stored on a suitable media, such as video recorder 360 or DVD/CD 370. Such information could assist in a variety of ways, such as education, patient billing, technical services or patient quality assurance. Providing a record of such information may also allow the surgeon to review a surgical procedure post operatively and thereby evaluate alternative surgical techniques and devices. Console 12 may also contain appropriate software that recognizes when console 12 requires service or repair and alerts automatically manufacturer 350.

In addition, system 10 allows for remote access to console 12 via hardware 300. Such remote access allows for the software used in console 12 to be updated remotely by manufacturer 350. In addition, service diagnoses made be made remotely. Training and other educational materials can be downloaded into console 12 and provided automatically whenever required or requested. Pre-operative patient data may be downloaded into console 12 so that the surgeon has a patient's complete medical record available during the surgical procedure. Package labelling, contraindications and directions for use for each surgical device to be used during the procedure, as well as the operation manual for console 12 can be downloaded onto console 12 and be available during the surgical procedure. Such information can be updated automatically by manufacturer 350.

This description is given for purposes of illustration and explanation. It will be apparent to those skilled in the relevant art that changes and modifications may be made to the invention described above without departing from its scope.

## Claims

1. A surgical procedure identification system (10), comprising:
a) a console (12) having a reader (21);
b) at least one surgical pak (14) containing a plurality of components (16);
c) an identification device (18) associated with the surgical pak, the identification device capable of being recognized by the reader for identifying the components contained in the pak to the console; and
d) an external device electronically connected (300) to the console for receiving information about the components contained in the pak;
**characterized in that** the console is a surgical console comprising a central processing unit (118) under the control of software adapted to;
use factory or user programmable settings to preprogram operating parameters in the surgical console for a specific surgeon or for a particular surgical procedure, comprising aspiration fluid flow rate and vacuum, irrigation fluid flow rate and pressure, and handpiece power and duty cycle,
read information identifying all of the components in a combination of components (16) comprising the sterile surgical pak (14), required to perform said particular surgical procedure;
adjust automatically the operating parameters of the surgical console to be used by the surgical console in said particular surgical procedure in response to said information to coincide with the components of the pak and said preprogrammed settings.

2. The surgical procedure identification system of claim 1, wherein the external device is a printer (220) for printing out a list (200) of the components (16) contained in the pak (14).

3. The surgical procedure identification system of claim 2, wherein the list (200) is a plurality of labels (210).

4. The surgical procedure identification system of claim 1, wherein the external device is an inventory management system (230) for tracking usage of the pak (14) and/or the components contained in the pak.

5. The surgical procedure identification system of any of claims 1 to 4, wherein the identification device (18) is a bar code and the reader (21) is a bar code reader.

6. The surgical procedure identification system of any of claims 1 to 5, wherein said electronic connection (300) between the surgical console (12) and the external device is a wireless connection.

7. The surgical procedure identification system of any of claims 1 to 6, wherein the surgical console (12) is capable of storing data, is electronically connected to a communications device, the device being located external to the surgical console, and is adapted to transfer data electronically between the surgical console and the device.

8. The surgical procedure identification system of claim 7, wherein the communications device is selected from any one of; a local area network server (310), a personal digital assistant (320), an internet server (330), an intranet server; and wherein said device communicates data to the surgical console.

9. Software for controlling a surgical procedure identification system as defined in claims 1-8 comprising;
program code adapted to use factory or user programmable settings to preprogram operating parameters in the surgical console for a specific surgeon or for a particular surgical procedure, comprising aspiration fluid flow rate and vacuum, irrigation fluid flow rate and pressure, and handpiece power and duty cycle,
program code adapted to read information identifying all of the components in a combination of components (16) comprising a sterile surgical pak (14), required to perform said particular surgical procedure;
program code adapted to adjust automatically the operating parameters of the surgical console to be used by the surgical console in a said particular surgical procedure in response to said information to coincide with the components of the pak and said preprogrammed settings.

10. Software according to claim 10, further comprising any one of;
program code for inventory management (230),
program code (300) allowing communication with devices (310,320,330) or a database (340) external to the console,
program code adapted to allows intercommunication with the console manufacturer (350).

## Patentansprüche

1. Chirurgisches System zur Vorgangs identifikation (10), das folgendes umfaßt:
a) eine Konsole (12) mit einem Lesegerät (21);
b) wenigstens ein chirurgisches Paket (14), das mehrere Komponenten (16) enthält;
c) eine Identifikationseinrichtung (18), die dem chirurgischen Paket zugeordnet ist, wobei die Identifikationseinrichtung in der Lage ist, von dem Lesegerät erkannt zu werden, um die Komponenten, die in dem Paket enthalten sind, für die Konsole zu identifizieren; und
d) eine externe Einrichtung, die elektronisch mit der Konsole verbunden ist (300), um Informationen über die Komponenten, die in dem Paket enthalten sind, zu empfangen;
**dadurch gekennzeichnet, daß** die Konsole eine chirurgische Konsole ist, die eine zentrale Bearbeitungseinheit (118) aufweist, die unter der Steuerung von Software steht, welche dafür eingerichtet ist:
hersteller- oder benutzerprogrammierbare Einstellungen zu verwenden, um Betriebsparameter in der chirurgischen Konsole für einen bestimmten Chirurgen oder für einen bestimmten chirurgischen Vorgang vorzuprogrammieren, die eine Strömungsrate und einen Unterdruck eines Aspirationsfluids, eine Strömungsrate und
einen Druck eines Spülfluids und eine Leistung und einen Arbeitszyklus eines Handstücks aufweisen,
Informationen zu lesen, welche alle der Komponenten identifizieren, die in Verbindung mit den im sterilen chirurgischen Paket (14) enthaltenen Komponenten (16) zum Ausführen des bestimmten chirurgischen Vorgangs erforderlich sind; und
die Betriebsparameter der chirurgischen Konsole automatisch einzustellen, die von der chirurgischen Konsole bei dem bestimmten chirurgischen Vorgang zu verwenden sind, in Reaktion auf die Information, um mit den Komponenten des Paktes und den vorprogrammierten Einstellung übereinzustimmen.

2. Chirurgisches System zur Vorgangs identifikation nach Anspruch 1, bei dem die externe Einrichtung ein Drucker (220) zum Ausdrucken einer Liste (200) der Komponenten (16) ist, die in dem Paket (14) enthalten sind.

3. Chirurgisches System zur Vorgangsidentifikation nach Anspruch 2, bei dem die Liste (200) eine Mehrzahl von Markierungen (210) ist.

4. Chirurgisches System zur Vorgangs identifikation nach Anspruch 1, bei dem die externe Einrichtung ein Bestandsverwaltungssystem (230) ist, um den Verbrauch des Pakets (14) und/oder der Komponenten, die in dem Paket enthalten sind, zu verfolgen.

5. Chirurgisches System zur Vorgangs identifikation nach einem der Ansprüche 1 bis 4, bei dem die Identifikationseinrichtung (18) ein Barcode ist und das Lesegerät (21) ein Barcode-Lesegerät ist.

6. Chirurgisches System zur Vorgangs identifikation nach einem der Ansprüche 1 bis 5, bei dem die elektronische Verbindung (300) zwischen der chirurgischen Konsole (12) und der externen Einrichtung eine drahtlose Verbindung ist.

7. Chirurgisches System zur Vorgangs identifikation nach einem der Ansprüche 1 bis 6, bei dem die chirurgische Konsole (12) in der Lage ist, Daten zu speichern, elektronisch mit einer Kommunikationseinrichtung verbunden ist, die Einrichtung außerhalb der chirurgischen Konsole angeordnet ist und dafür eingerichtet ist, Daten elektronisch zwischen der chirurgischen Konsole und der Einrichtung zu übertragen.

8. Chirurgisches System zur Vorgangs identifikation nach Anspruch 7, bei dem die Kommunikationseinrichtung aus einem von folgendem ausgewählt ist: ein Local-Area-Network-Server (310), ein Personal-Digital-Assistant (320), ein Internetserver (330), ein Intranetserver; und bei dem die Einrichtung Daten zu der chirurgischen Konsole überträgt.

9. Software zum Steuern eines chirurgischen Systems zur Vorgangs identifikation, wie in den Ansprüchen 1 bis 8 definiert, die folgendes aufweist:
Programmcode, der dafür eingerichtet ist, hersteller- oder benutzerprogrammierbare Einstellungen zu verwenden, um Betriebsparameter in der chirurgischen Konsole für einen bestimmten Chirurgen oder für einen bestimmten chirurgischen Vorgang vorzuprogrammieren, die eine Strömungsrate und einen Unterdruck eines Aspirationsfluids, eine Strömungsrate und einen Druck eines Spülfluids und eine Leistung und einen Arbeitszyklus eines Handstücks umfassen,
Programmcode, der dafür eingerichtet ist, Informationen zu lesen, welche alle der Komponenten identifizieren, die in Verbindung mit in einer sterilen chirurgischen Packung (14) enthaltenen Komponenten (16) zum Ausführen des bestimmten chirurgischen Vorgangs erforderlich sind; und
Programmcode, der dafür eingerichtet ist, die Betriebsparameter der chirurgischen Konsole automatisch einzustellen, die von der chirurgischen Konsole bei dem bestimmten chirurgischen Vorgang zu verwenden sind, in Reaktion auf die Information, um mit den Komponenten des Pakets und den vorprogrammierten Einstellungen übereinzustimmen

10. Software nach Anspruch 10, die ferner eines von folgendem aufweist:
Programmcode für eine Bestandsverwaltung (230),
Programmcode (300), der eine Verbindung mit Einrichtungen (310, 320, 330) oder einer Datenbank (340) außerhalb der Konsole ermöglicht, und
Programmcode, der dafür eingerichtet ist, einen Wechselverkehr mit dem Konsolenhersteller (350) zu ermöglichen.

## Revendications

1. Système d'identification d'opération chirurgicale (10), comportant :
a) une console (12) ayant un lecteur (21),
b) au moins une plaquette chirurgicale (14) contenant une pluralité de composants (16),
c) un dispositif d'identification (18) associé à la plaquette chirurgicale, le dispositif d'identification pouvant être reconnu par le lecteur pour identifier les composants contenus dans la plaquette de la console, et
d) un dispositif externe électroniquement relié (300) à la console pour recevoir des informations concernant les composants contenus dans la plaquette,
**caractérisé en ce que** la console est une console chirurgicale comportant une unité centrale de traitement (118) sous la commande d'un logiciel adapté pour :
utiliser des réglages programmables en usine ou par l'utilisateur pour préprogrammer des paramètres de fonctionnement de la console chirurgicale pour un chirurgien spécifique ou pour une opération chirurgicale particulière, comportant le débit d'écoulement de fluide et de vide d'aspiration, le débit et la pression d'écoulement de fluide d'irrigation, et la puissance et le rapport cyclique d'une pièce à main,
lire des informations identifiant tous les composants d'une combinaison de composants (16) constituant la plaquette chirurgicale stérile (14), requises pour réaliser ladite opération chirurgicale particulière,
régler automatiquement les paramètres de fonctionnement de la console chirurgicale pour être utilisés par la console chirurgicale dans ladite opération chirurgicale particulière en réponse auxdites informations de manière à coïncider avec les composants de la plaquette et lesdits réglages préprogrammés.

2. Système d'identification d'opération chirurgicale selon la revendication 1, dans lequel le dispositif externe est une imprimante (220) pour imprimer une liste (200) des composants (16) contenus dans la plaquette (14).

3. Système d'identification d'opération chirurgicale selon la revendication 2, dans lequel la liste (200) est une pluralité d'étiquettes (210).

4. Système d'identification d'opération chirurgicale selon la revendication 1, dans lequel le dispositif externe est un système de gestion de stocks (230) pour suivre l'utilisation de la plaquette (14) et/ou des composants contenus dans la plaquette.

5. Système d'identification d'opération chirurgicale selon l'une quelconque des revendications 1 à 4, dans lequel le dispositif d'identification (18) est un code barres et le lecteur (21) est un lecteur de code barres.

6. Système d'identification d'opération chirurgicale selon l'une quelconque des revendications 1 à 5, dans lequel ladite connexion électronique (300) entre la console chirurgicale (12) et le dispositif externe est une connexion sans fil.

7. Système d'identification d'opération chirurgicale selon l'une quelconque des revendications 1 à 6, dans lequel la console chirurgicale (12) est capable de mémoriser des données, est électroniquement connectée à un dispositif de communications, le dispositif étant placé à l'extérieur de la console chirurgicale, et est adapté pour transférer électroniquement des données entre la console chirurgicale et le dispositif.

8. Système d'identification d'opération chirurgicale selon la revendication 7, dans lequel le dispositif de communications est sélectionné parmi l'un quelconque d'un serveur de réseau local (310), un assistant numérique personnel (320), un serveur Internet (330), un serveur intranet, et dans lequel ledit dispositif communique des données à la console chirurgicale.

9. Logiciel pour commander un système d'identification d'opération chirurgicale selon les revendications 1 à 8, comportant :
un code de programme adapté pour utiliser des réglages programmables en usine ou par l'utilisateur pour préprogrammer des paramètres de fonctionnement de la console chirurgicale pour un chirurgien spécifique ou pour une opération chirurgicale particulière, comportant le débit d'écoulement de fluide et de vide d'aspiration, le débit et la pression d'écoulement de fluide d'irrigation, et la puissance et le rapport cyclique d'une pièce à main,
un code de programme adapté pour lire des informations identifiant tous les composants dune combinaison de composants (16) constituant une plaquette chirurgicale stérile (14), requises pour réaliser ladite opération chirurgicale particulière,
un code de programme adapté pour régler automatiquement les paramètres de fonctionnement de la console chirurgicale pour être utilisés par la console chirurgicale dans ladite opération chirurgicale particulière en réponse auxdites informations pour coïncider avec les composants de la plaquette et lesdits réglages préprogrammés.

10. Logiciel selon la revendication 9, comportant de plus l'un quelconque parmi :
un code de programme pour la gestion de stocks (230),
un code de programme (300) permettant la communication avec des dispositifs (310, 320, 330) ou une base de données (340) à l'extérieur de la console,
un code de programme adapté pour permettre une intercommunication avec le fabricant de console (350).
